# EUROPEAN PATENT SPECIFICATION

(11) **EP 4 380 428 B1**
(45) Date of publication and mention of the grant of the patent: **11.11.2026**
(21) Application number: 22748038.1
(22) Date of filing: 12.07.2022
(51) Int. Cl.: A61B 5/00, A61B 5/257, A61B 5/259, A61B 5/282, A61B 5/283

(54) **SENSING SYSTEM FOR SENSING BIOLOGICAL SIGNALS EXTERNALLY ON A PATIENT**
SENSORSYSTEM ZUR EXTERNEN ERFASSUNG VON BIOLOGISCHEN SIGNALEN AN EINEM PATIENTEN
SYSTÈME DE DÉTECTION DE SIGNAUX BIOLOGIQUES EXTERNES SUR UN PATIENT

(30) Priority: 06.08.2021 US 202163230211 P; 07.09.2021 EP 21195179
(43) Date of publication of application: 12.06.2024
(73) Proprietor: BIOTRONIK SE & Co. KG, 12359 Berlin (DE)
(72) Inventor: GUHANIYOGI, Shayan, Portland, Oregon 97206 (US); MELIUS, Matthew, Milwauki, Oregon 97267 (US); AUSTIN, Eric, Portland, Oregon 97221 (US); KRAETSCHMER, Hannes, West Linn, Oregon 97068 (US); WHITTINGTON, R. Hollis, Portland, Oregon 97202 (US); MUESSIG, Dirk, West Linn, Oregon 97068 (US)
(74) Representative: Biotronik Corporate Services SE
(86) International application number: PCT/EP2022/069405
(87) International publication number: WO 2023/011870

(56) References cited:
- US-A- 6 117 077
- US-A1- 2007 270 678
- US-A1- 2012 089 037
- US-A1- 2020 315 489

## Description

The invention generally relates to a sensing system, according to independent claim 1, for sensing biological signals externally on a patient.

The provision and the use of such a system are defined in independent claims 14 and 15, respectively.

Using a sensing system of the kind concerned herein biological signals shall be sensed outside of a patient. In particular, a sensing system of this kind may serve to sense electrocardiogram (ECG) signals, wherein for this contact elements for example in the shape of surface ECG electrodes may be attached to the patient's skin in order to make electrical contact to the patient, such that signals may be monitored on the patient.

Generally, different approaches exist to monitor biological signals, in particular cardiac signals such as electrocardiogram signals, on a patient.

If a short-term monitoring is required, a monitoring device may be attached externally to a patient in order to sense biological signals on the patient, for example over a duration of multiple days or a few weeks.

If a long-term monitoring shall be conducted, an implantable medical device in the shape of a biological sensor may be implanted in a patient, in particular subcutaneously within a minimally invasive procedure. An implantable medical device of this kind may remain within the patient over a rather long duration, for example several months or even years, such that signals may be sensed in the context of a long-term monitoring, for example to detect cardiac arrhythmias or the like.

There is a general desire to provide for a short-term sensing system which may be easily applied to a patient and may be comfortably worn externally by the patient.

It is an object of the instant invention to provide a sensing system and a method for providing a sensing system which allow, in an easy and cost-efficient manner, for a sensing of biological signals externally on a patient, while achieving a reliable monitoring as well as an increased comfort for a patient.

In one aspect, a sensing system for sensing biological signals externally on a patient comprises: an implantable medical device comprising at least one electrode for sensing biological signals on a patient; an adapter element mountable on said at least one electrode to electrically contact with the at least one electrode; a fastening component mounted or mountable on said adapter element and configured to attach the medical device externally to the patient or patient's body; a contact element mounted or mountable to the fastening component and configured to attach externally to the patient's skin for receiving biological signals and conducting said biological signals to the adapter element; and a casing for encasing the medical device and the adapter element in a mounted state in which the adapter element is mounted on said at least one electrode.

The fastening component may comprise a contact face configured to touch or contact the patient's skin. The contact element is preferably attachable/mountable or configured to be attached/mounted to the contact face of the fastening component.

In order to be able to transmit or conduct the biological signals from the contact element to the at least one electrode, the adapter element, the fastening component and/or the contact element may be electrically conductive or at least partially made of an electrically conductive material.

The medical device is designed for implantation into a patient. Within the implantable medical device, the at least one electrode may be configured to electrically contact biological tissue to sense biological signals in an implanted state of the medical device.

For example, the medical device may be a biosensor which originally is designed for subcutaneous implantation into a patient for sensing electrocardiogram (ECG) signals.

Within the sensing system, a medical device - which is originally designed for implantation into a patient - is used for an external sensing of biological signals, in particular cardiac signals, such as electrocardiogram signals. In particular, the medical device may be an implantable loop recorder or implantable heart monitor. For this, the medical device is combined with one or multiple adapter elements, each of which contains or is connected to a fastening component. The fastening component preferably fastens to contact elements that are attached externally on the patient's skin in order to pick up biological signals on the patient and to conduct the biological signals via the fastening component of the adapter element to the at least one electrode of the medical device.

It generally can be assumed that an implantable sensor device comprises a processing circuitry which makes the device usable also for an external monitoring. Hence, by means of the instant sensing system it is proposed to use the medical device, which originally may not have been designed for an external use, for an external monitoring by adapting the medical device for an external use using one or multiple adapter elements and one or multiple contact elements.

Within an implantable medical device the at least one electrode is designed to electrically contact with surrounding tissue when the implantable medical device is implanted in a patient. However, an electrode of this kind may as such not be suitable when using the implantable medical device externally on a patient. Therefore, an adapter element is used to engage with the at least one electrode of the medical device in order to make electrical contact to the at least one electrode, wherein an attachment on the patient is achieved by means of the fastening component and/or the contact element. At least the fastening component is in contact with the adapter element and hence may pick up biological signals from the patient's skin and/or the contact element for conducting the biological signals via the adapter element towards the at least one electrode. It shall be noted that the contact element is not necessarily a part of the system. For example, the fastening component may be attached directly to the patient's skin and made of an electrically conductive material. Thus, the system could function equally or at least similarly even without the contact element. However, the system could function also equally or at least similarly without the fastening component, wherein the adapter element is connected/connectable directly to the contact element. Alternatively, the fastening component may be a part of the adapter element.

The contact element may have the shape of a commercially available ECG electrode which may be adhesively attached to a patient.

The fastening component and/or the contact element, in one embodiment, is releasably connectable to the adapter element. For example, the fastening component and/or the adapter element may comprise a button connector which allows for an attachment of the fastening component and/or the adapter element to the contact element, wherein the fastening component and/or the adapter element may be attachable to different contact elements, in particular contact elements of different size.

The adapter element and/or the fastening component is/are electrically conductive such that biological signals may be conducted from the contact element towards the at least one electrode of the medical device for processing the biological signals within the medical device.

The assembly of the adapter element and the medical device is encased in a casing, which preferably is made from a biocompatible material. The medical device with the adapter element mounted thereon hence is received within a protective casing, which is placed externally on a patient and protects the medical device with the adapter element arranged thereon towards the outside.

In one embodiment, at least one of the medical device and the adapter element (preferably including the fastening component) are removable from the casing. This allows for a reuse of the casing as well as the medical device and the adapter element received therein. For example, by removing the medical device and the adapter element from the casing, the medical device as well as the adapter element and the casing may be thoroughly cleaned, such that the casing, the medical device and the adapter element may be reused on another patient. Also, whereas the medical device and/or the adapter element may be designed for a reuse, the casing may be designed only for a one-time usage, in which case the casing may be discarded after use and after removal of the medical device and the adapter element received therein.

In one embodiment, the adapter element is releasably mounted on the at least one electrode. In particular, the mounting of the adapter element on the medical device may be such that no modification to the medical device is required. By hence placing the adapter element on the medical device, an electrical contact in between the adapter element and the at least one electrode of the medical device can be established, wherein after use the adapter element may be again removed from the medical device in order to for example prepare the medical device and/or the adapter element for a reuse, for example by cleaning the medical device and the adapter element.

In one embodiment, the adapter element may be fixed on the medical device for example using a screw or the like.

In an embodiment in which the adapter element is to be non-removably connected to the medical device and/or the at least one electrode, for example a welding connection in between the adapter element and the medical device may be established.

In one embodiment, the adapter element comprises a body forming a reception opening for receiving the at least one electrode therein. By inserting the medical device with an electrode arranged thereon into the reception opening of the body of the adapter element an electrical contact in between the adapter element and the electrode may be established. This allows for an easy mounting of the adapter element on the medical device, and in addition a reliable operation, in particular a reliable electrical contact in between the adapter element and the electrode of the medical device.

In one embodiment, the medical device comprises a housing forming a main housing portion and a header portion extending from the main housing portion. An electronics module may for example be arranged in or on the main housing portion, whereas for example one or multiple electrodes are arranged in or on the header portion. The header portion may for example extend linearly from the main housing portion, wherein the header portion may be flexibly deformable, or may have a rigid structure such that it is not flexibly deformable.

Herein, a first electrode may for example be arranged on an end of the header portion which is distal with respect to the main housing portion. Another, second electrode may, in one embodiment, be placed on the main housing portion, for example at an end of the main housing portion opposite to the header portion. Each electrode herein may be associated with a dedicated adapter element, such that the first electrode is electrically in contact with a first adapter element connected to a first contact element and/or a first fastening component, and the second electrode is electrically in contact with another, second adapter element connected to a second contact element and/or a second fastening component, such that each electrode is attached to the patient for sensing biological signals on the patient.

In one embodiment, the adapter element and the fastening element are formed in one piece. This simplifies the structure and assembly of the system.

In another embodiment, the adapter element, the fastening element and the contact element are formed in one piece. This particularly simplifies the structure and assembly of the system.

Additionally or alternatively, at least two of the adapter element, the fastening element and the contact element may be connectable to each other via button connectors.

In another aspect, a method for providing a sensing system for sensing biological signals externally on a patient comprises: providing an implantable medical device comprising at least one electrode for sensing biological signals on a patient; mounting an adapter element on said at least one electrode to electrically contact with the at least one electrode; providing a fastening component mounted or mountable on said adapter element (3A, 3B) and configured to attach the medical device (1) externally to the patient; providing a contact element mounted or mountable to the fastening component and configured to attach externally to the patient's skin for receiving biological signals and conducting said biological signals to the adapter element; and encasing, using a casing, the medical device and the adapter element in a mounted state in which the adapter element is mounted on said at least one electrode.

In yet another aspect, a sensing system is used for sensing biological signals externally on a patient, the sensing system comprising: an implantable medical device comprising at least one electrode for sensing biological signals on a patient; an adapter element mountable on said at least one electrode to electrically contact with the at least one electrode; a fastening component (4) mounted or mountable on said adapter element (3A, 3B) and configured to attach the medical device (1) externally to the patient; a contact element mounted or mountable to the fastening component and configured to attach externally to the patient's skin for receiving biological signals and conducting said biological signals to the adapter element; and a casing for encasing the medical device and the adapter element in a mounted state in which the adapter element is mounted on said at least one electrode.

The advantages and advantageous embodiments described above for the sensing system equally apply also to the method and the use, such that it shall be referred to the above in this respect.

By using an implantable medical device in combination with one or multiple adapter elements and one or multiple contact elements for electrically attaching one or multiple electrodes of the implantable medical device externally to the patient's skin, an implantable device which as such is designed for an implanted use may be used as an external monitoring device. By encasing the arrangement of the medical device and one or multiple adapter elements attached to the medical device a functional module is provided which may be placed outside on a patient and may be slender in build.

The various features and advantages of the present invention may be more readily understood with reference to the following detailed description and the embodiments shown in the drawings. Herein,
- Fig. 1: shows a schematic drawing of an implantable medical device implanted in a patient;
- Fig. 2: shows a schematic view of an embodiment of an implantable medical device together with an external communication device;
- Fig. 3: shows a view of an implantable medical device comprising a first electrode on a header portion and a second electrode on a main body portion;
- Fig. 4: shows an adapter element to be mounted on an electrode of the medical device, including the fastening component, in combination with a contact element to be attached to a patient;
- Fig. 5: shows the medical device of Fig. 3 together with two adapter elements (including fastening component) for attaching the medical device to a patient;
- Fig. 6: shows the arrangement of Fig. 5 encased in a casing; and
- Fig. 7: shows the arrangement of Fig. 6 in a semitransparent view of the casing.

Subsequently, embodiments of the invention shall be described in detail with reference to the drawings. In the drawings, like reference numerals designate like structural elements.

It is to be noted that the embodiments are not limiting for the invention, but merely represent illustrative examples. It is understood that the invention is solely defined by the appended claims.

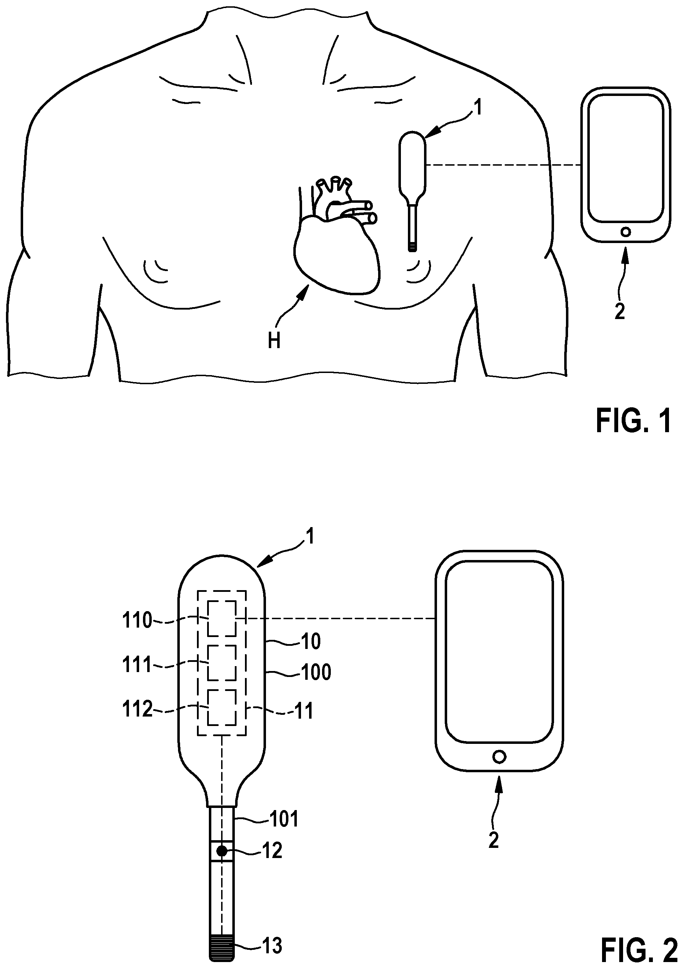

Within the instant text it is proposed to use an implantable medical device 1, as shown in one embodiment in Figs. 1 and 2, externally on a patient to provide for a sensing system for an external monitoring and sensing of biological signals.

Referring to Fig. 1, in one embodiment an implantable medical device 1 is designed for implantation (for example subcutaneous implantation) into a patient. The implantable medical device 1 may for example be a sensor device for measuring biological signals and may be designed to be implanted subcutaneously within a patient in proximity to the patient's heart H, for example left-pectorally, in order to sense biological signals from the patient's heart H, such as electrocardiogram (ECG) signals. An exemplary insertable cardiac monitor intended for subcutaneous implantation is known from US 2020/315489 A1.

In an implanted state, the implantable medical device 1 e.g. shall sense signals and shall transfer information relating to sensed signals to an external communication device 2, for example within a home monitoring system. For this, the implantable medical device 1 may employ a standardized communication technology, such as the Zigbee technology or the Bluetooth Low Energy (BLE) technology.

Referring now to Fig. 2, in one embodiment the implantable medical device 1 comprises a housing 10 forming a main housing portion 100 and a header portion 101 linearly extending from the main housing portion 100. An electronics module 11 is arranged within the main housing portion 100, the electronics module 11 forming a communication circuitry 110 for establishing a communication with an external communication device 2. Further, the electronics module 11 comprises a processing circuitry 112 for processing signals for stimulation or sensing, and an energy storage 111 for supplying energy in the shape of a battery for operating the implantable medical device 1.

The header portion 101, in the embodiment of Fig. 2, comprises an electrode arrangement 13 comprising an electrode which is arranged at a distal end of the header portion 101 and hence at a remote location with respect to the electronics module 11 arranged within the main housing portion 100. In addition, an antenna 12 is formed on the header portion 101, the antenna 12 serving to transmit and/or receive signals, in cooperation with the communication circuitry 110, for communication with the external communication device 2.

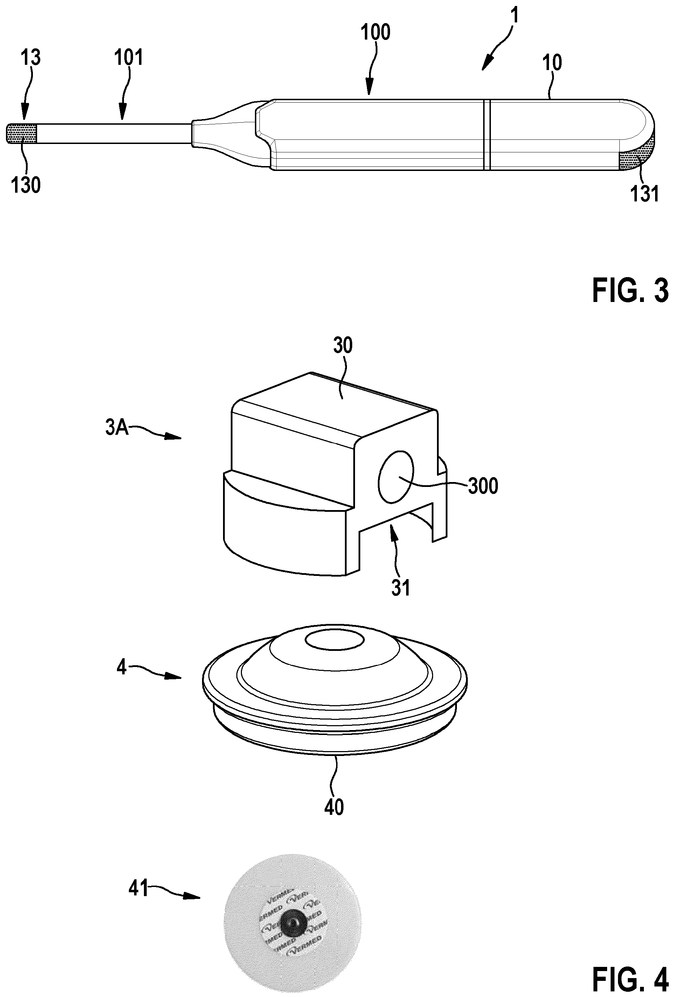

Referring now to Figs. 3 to 5, in one embodiment the implantable medical device 1 comprises two electrodes 130, 131 with a first electrode 130 and a second electrode 131 together forming an electrode arrangement 13 for sensing biological signals, in particular electrocardiogram signals. The electrodes 130, 131 herein are designed particularly for sensing signals in an implanted state of the implantable medical device 1 by making electrical contact to tissue surrounding the implantable medical device 1.

As it is visible from Fig. 3, a medical device 1 designed for implantation generally is slender in built and comprises a minimized form factor. A medical device 1, for example in the shape of a biosensor for sensing electrocardiogram signals, generally comprises circuitry which however makes the medical device 1 suitable also for an external use to sense biological signals, such as electrocardiogram signals, externally on a patient.

It hence, in the context of this text, is proposed to use a medical device 1 which originally is designed for a different purpose, such as for implantation, for an external sensing by adapting the medical device 1 for an external use.

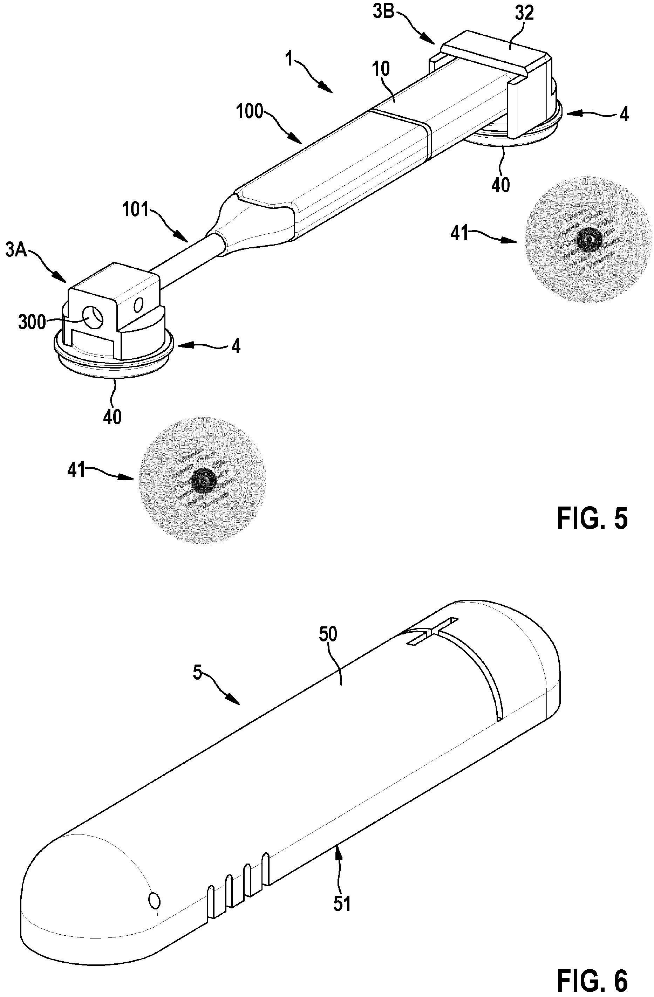

For this, adapter elements 3A, 3B are provided, as shown in Figs. 4 and 5, which are associated with the electrodes 130, 131 of the medical device 1 and may be mounted on the medical device 1 such that the adapter elements 3A, 3B make electrical contact with the electrodes 130, 131.

For example, an adapter element 3A adapted to contact the electrode 130 on the header portion 101 of the housing 10 of the medical device 1 may comprise a body 30 forming a reception opening 300 in the shape of a through-hole into which the header portion 101 may be inserted in order to bring the electrode 130 arranged distally on the header portion 101 into electrical contact with the body 30 of the adapter element 3A.

An adapter element 3B associated with the electrode 131 arranged on the main housing portion 100, in turn, may comprise a box-like body 32 in which the main housing portion 100 with an end remote from the header portion 101 may be received, such that the electrode 131 electrically contacts with the body 32.

Each of the adapter elements 3A, 3B herein includes or is connectable to a fastening component 4 in the shape of for example a button connector. The fastening component enables releasably connecting the adapter elements 3A, 3B to contact elements 41, by means of contact faces 40, such that the contact elements 41 electrically contact with the patient and in addition are in electrical contact with the adapter elements 3A, 3B via the fastening components 4, and, via the adapter elements 3A, 3B, with the electrodes 130, 131 of the electrode arrangement 13 of the medical device 1. The contact elements 41 may be for example commercially available surface ECG electrodes which may be adhesively attached to the patient's skin. Each of the adapter elements 3A, 3B may for example comprise a fastening device 31 for example in the shape of a button connector, such that the adapter elements 3A, 3B may be releasably connected to the contact elements 41 and/or to the fastening components 4.

The contact elements 41 (e.g. by means of a contact face 40) may be placed on the patient's skin and may be adhesively attached to the patient, such that the contact elements 41 electrically contact with the patient and in addition are in electrical contact with the adapter elements 3A, 3B and/or fastening components 4 and, via the adapter elements 3A, 3B, with the electrodes 130, 131 of the electrode arrangement 13 of the medical device 1.

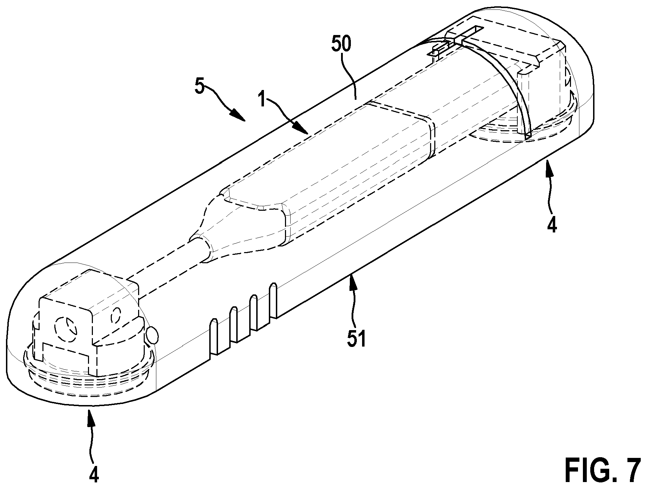

Referring now to Figs. 6 and 7, the assembly of Fig. 5 may be received within a casing 5 in order to provide for a modular device which may be placed and used on a patient. The casing 5 herein comprises an outer housing wall 50 which provides for a protective shielding towards the outside. The casing 5 may also be called external casing 5 or patch 5. By means of a bottom face 51 the casing 5 may be placed on a patient, wherein the fastening components 4 of the adapter elements 3A, 3B, are accessible at the bottom face 51 and hence may be brought into contact with contact elements 41 on the patient's skin by placing the casing 5 on the patient and/or the contact elements 41.

The casing 5 may for example be made from a biocompatible material. In that the casing 5 is adapted to the slender shape of the medical device 1, the sensing system as provided by the arrangement of Figs. 6 and 7 is slender in built and hence comfortable to wear for a patient.

The casing 5 may comprise an opening, or may be adapted for disassembly, for removing the medical device 1 and the adapter elements 3A, 3B, for example including the fastening components 4, from the casing 5. In addition, the adapter elements 3A, 3B may be removable from the medical device 1, such that the sensing system may fully be disassembled. This allows for a reuse of the casing 5, the medical device 1 and/or the adapter elements 3A, 3B, wherein for a reuse the components of the system may be disassembled and thoroughly cleaned and may subsequently be reused on a different patient.

In one embodiment, the medical device 1, the adapter elements 3A, 3B (e.g. including fastening components 4) and the casing 5 are reusable.

In another embodiment, only the medical device 1 and the adapter elements 3A, 3B (e.g. including fastening components 4) are reusable, whereas the casing 5 is to be disposed after a one-time use.

The implantable medical device 1 may be usable within the sensing system in a complete production state in which it also is usable as an implantable medical device for implantation into a patient.

In another embodiment, the medical device 1 is not in a complete production state when used in the sensing system for external sensing, but for example only components, such as an electrode arrangement 13 and an electronics module 11 e.g. on a circuit board, are used for the sensing system and for this are encased in a casing 5.

The contact elements 41 and/or the fastening components 4 are removable from the adapter elements 3A, 3B, wherein the contact elements 41 and/or the fastening components 4 of arbitrary shape may be selected for use in the sensing system. The contact elements 41 and or the fastening components 4 may for example be or compatible with commercially available surface ECG electrodes.

The invention is defined by the appended claims.

### List of reference numerals

- 1: (Implantable) medical device
- 10: Housing
- 100: Main housing portion
- 101: Header portion
- 11: Electronics module
- 110: Communication circuitry
- 111: Energy storage
- 112: Processing circuitry
- 12: Antenna
- 13: Electrode arrangement
- 130, 131: Electrode
- 2: External communication device
- 3A, 3B: Adapter element
- 30: Body (of adapter element 3A)
- 300: Reception opening
- 31: Fastening device
- 32: Body (of adapter element 3B)
- 4: Fastening component
- 40: Contact face
- 41: Contact element
- 5: Casing
- 50: Housing wall
- 51: Bottom face
- H: Heart

## Claims

1. A sensing system for sensing biological signals externally on a patient, comprising:
an implantable medical device (1) comprising at least one electrode (130, 131) for sensing biological signals on a patient;
an adapter element (3A, 3B) mountable on said at least one electrode (130, 131) to electrically contact with the at least one electrode (130, 131);
a fastening component (4) mountable on said adapter element (3A, 3B) and configured to attach the medical device (1) externally to the patient;
a contact element (41) mountable to the fastening component (4) and configured to attach externally to the patient's skin for receiving biological signals and conducting said biological signals to the adapter element (3A, 3B); and
a casing (5) for encasing the medical device (1) and the adapter element (3A, 3B) in a mounted state in which the adapter element (3A, 3B) is mounted on said at least one electrode (130, 131).

2. The sensing system according to claim 1, wherein the medical device (1) is implantable into a patient, said at least one electrode (130, 131) being configured to electrically contact biological tissue to sense biological signals in an implanted state of the medical device (1).

3. The sensing system according to claim 1 or 2, wherein the medical device (1) is a bio-sensor for sensing electrocardiogram signals.

4. The sensing system according to one of claims 1 to 3, wherein the fastening component (4) is releasably connectable to said adapter element (3A, 3B).

5. The sensing system according to one of the preceding claims, wherein at least one of the medical device (1) and the adapter element (3A, 3B) are removable from the casing (5).

6. The sensing system according to one of the preceding claims, wherein the adapter element (3A, 3B) is releasably mountable on said at least one electrode (130, 131).

7. The sensing system according to one of the preceding claims, wherein the adapter element (3A, 3B) comprises a body (30) forming a reception opening (300) for receiving said at least one electrode (130, 131) therein.

8. The sensing system according to one of the preceding claims, wherein the medical device (1) comprises a housing (10) forming a main housing portion (100) and a header portion (101) extending from the main housing portion (100), and an electronics module (11) being arranged in or on the main housing portion (100).

9. The sensing system according to claim 8, wherein the header portion (101) linearly extends from the main housing portion (100).

10. The sensing system according to claim 8 or 9, wherein a first electrode (130) is placed on the header portion (101) and a second electrode (131) is placed on the main housing portion (100).

11. The sensing system according to claim 10, wherein an adapter element (3A, 3B) is mountable on each of the first electrode (130) and the second electrode (131).

12. The sensing system according to one of the preceding claims, wherein the adapter element (3A, 3B) and the fastening element (4) are formed in one piece.

13. The sensing system according to one of the preceding claims, wherein the adapter element (3A, 3B), the fastening element (4) and the contact element (41) are formed in one piece.

14. A method for providing a sensing system for sensing biological signals externally on a patient, comprising:
providing an implantable medical device (1) comprising at least one electrode (130, 131) for sensing biological signals on a patient;
mounting an adapter element (3A, 3B) on said at least one electrode (130, 131) to electrically contact with the at least one electrode (130, 131);
providing a fastening component (4) mountable on said adapter element (3A, 3B) and configured to attach the medical device (1) externally to the patient;
providing a contact element (41) mountable to the fastening component and configured to attach externally to the patient's skin for receiving biological signals and conducting said biological signals to the adapter element (3A, 3B); and
encasing, using a casing (5), the medical device (1) and the adapter element (3A, 3B) in a mounted state in which the adapter element (3A, 3B) is mounted on said at least one electrode (130, 131).

15. A use of a sensing system for sensing biological signals externally on a patient, the sensing system comprising:
an implantable medical device (1) comprising at least one electrode (130, 131) for sensing biological signals on a patient;
an adapter element (3A, 3B) mountable on said at least one electrode (130, 131) to electrically contact with the at least one electrode (130, 131);
a fastening component (4) mountable on said adapter element (3A, 3B) and configured to attach the medical device (1) externally to the patient;
a contact element (41) mountable to the fastening component and configured to attach externally to the patient's skin for receiving biological signals and conducting said biological signals to the adapter element (3A, 3B); and
a casing (5) for encasing the medical device (1) and the adapter element (3A, 3B) in a mounted state in which the adapter element (3A, 3B) is mounted on said at least one electrode (130, 131).

## Patentansprüche

1. Erfassungssystem zum externen Erfassen biologischer Signale an einem Patienten, umfassend:
ein implantierbares medizinisches Gerät (1), die mindestens eine Elektrode (130, 131) zum Erfassen biologischer Signale an einem Patienten umfasst;
ein Adapterelement (3A, 3B), das an der mindestens einen Elektrode (130, 131) montierbar ist, um die mindestens eine Elektrode (130, 131) elektrisch zu kontaktieren;
eine Befestigungskomponente (4), die an dem Adapterelement (3A, 3B) montierbar ist und dazu konfiguriert ist, das medizinische Gerät (1) extern an dem Patienten zu befestigen;
ein Kontaktelement (41), das an der Befestigungskomponente (4) montierbar ist und dazu konfiguriert ist, extern an der Haut des Patienten befestigt zu werden, um biologische Signale zu empfangen und diese biologischen Signale zu dem Adapterelement (3A, 3B) zu leiten; und
eine Einfassung (5) zum Einfassen des medizinischen Geräts (1) und des Adapterelements (3A, 3B) in einem montierten Zustand, in dem das Adapterelement (3A, 3B) an der mindestens einen Elektrode (130, 131) montiert ist.

2. Erfassungssystem nach Anspruch 1, wobei das medizinische Gerät (1) in einen Patienten implantierbar ist, wobei die mindestens eine Elektrode (130, 131) dazu konfiguriert ist, biologisches Gewebe elektrisch zu kontaktieren, um biologische Signale in einem implantierten Zustand des medizinischen Geräts (1) zu erfassen.

3. Erfassungssystem nach Anspruch 1 oder 2, wobei das medizinische Gerät (1) ein Biosensor zum Erfassen von Elektrokardiogrammsignalen ist.

4. Erfassungssystem nach einem der Ansprüche 1 bis 3, wobei die Befestigungskomponente (4) lösbar mit dem Adapterelement (3A, 3B) verbindbar ist.

5. Erfassungssystem nach einem der vorstehenden Ansprüche, wobei mindestens eines von dem medizinischen Gerät (1) und dem Adapterelement (3A, 3B) aus der Einfassung (5) entfernbar ist.

6. Erfassungssystem nach einem der vorstehenden Ansprüche, wobei das Adapterelement (3A, 3B) lösbar an der mindestens einen Elektrode (130, 131) montierbar ist.

7. Erfassungssystem nach einem der vorstehenden Ansprüche, wobei das Adapterelement (3A, 3B) einen Körper (30) umfasst, der eine Aufnahmeöffnung (300) zum Aufnehmen der mindestens einen Elektrode (130, 131) darin bildet.

8. Erfassungssystem nach einem der vorstehenden Ansprüche, wobei das medizinische Gerät (1) ein Gehäuse (10) umfasst, das einen Hauptgehäuseabschnitt (100) und einen sich von dem Hauptgehäuseabschnitt (100) erstreckenden Kopfabschnitt (101) bildet, und wobei ein Elektronikmodul (11) in oder an dem Hauptgehäuseabschnitt (100) angeordnet ist.

9. Erfassungssystem nach Anspruch 8, wobei sich der Kopfabschnitt (101) linear von dem Hauptgehäuseabschnitt (100) erstreckt.

10. Erfassungssystem nach Anspruch 8 oder 9, wobei eine erste Elektrode (130) auf dem Kopfabschnitt (101) angeordnet ist und eine zweite Elektrode (131) auf dem Hauptgehäuseabschnitt (100) angeordnet ist.

11. Erfassungssystem nach Anspruch 10, wobei auf jeder der ersten Elektrode (130) und der zweiten Elektrode (131) ein Adapterelement (3A, 3B) montierbar ist.

12. Erfassungssystem nach einem der vorstehenden Ansprüche, wobei das Adapterelement (3A, 3B) und das Befestigungselement (4) einstückig ausgebildet sind.

13. Erfassungssystem nach einem der vorstehenden Ansprüche, wobei das Adapterelement (3A, 3B), das Befestigungselement (4) und das Kontaktelement (41) einstückig ausgebildet sind.

14. Verfahren zum Bereitstellen eines Erfassungssystems zum externen Erfassen biologischer Signale an einem Patienten, umfassend:
Bereitstellen eines implantierbaren medizinischen Geräts (1), die mindestens eine Elektrode (130, 131) zum Erfassen biologischer Signale an einem Patienten umfasst;
Montieren eines Adapterelements (3A, 3B) an der mindestens einen Elektrode (130, 131), um die mindestens eine Elektrode (130, 131) elektrisch zu kontaktieren;
Bereitstellen einer Befestigungskomponente (4), die an dem Adapterelement (3A, 3B) montierbar ist und dazu konfiguriert ist, das medizinische Gerät (1) extern an dem Patienten zu befestigen;
Bereitstellen eines Kontaktelements (41), das an der Befestigungskomponente montierbar ist und dazu konfiguriert ist, extern an der Haut des Patienten befestigt zu werden, um biologische Signale zu empfangen und diese biologischen Signale zu dem Adapterelement (3A, 3B) zu leiten; und
Einfassen des medizinischen Geräts (1) und des Adapterelements (3A, 3B) unter Verwendung einer Einfassung (5) in einem montierten Zustand, in dem das Adapterelement (3A, 3B) an der mindestens einen Elektrode (130, 131) montiert ist.

15. Verwendung eines Erfassungssystems zum externen Erfassen biologischer Signale an einem Patienten, wobei das Erfassungssystem umfasst:
ein implantierbares medizinisches Gerät (1), die mindestens eine Elektrode (130, 131) zum Erfassen biologischer Signale an einem Patienten umfasst;
ein Adapterelement (3A, 3B), das an der mindestens einen Elektrode (130, 131) montierbar ist, um die mindestens eine Elektrode (130, 131) elektrisch zu kontaktieren;
eine Befestigungskomponente (4), die an dem Adapterelement (3A, 3B) montierbar ist und dazu konfiguriert ist, das medizinische Gerät (1) extern an dem Patienten zu befestigen;
ein Kontaktelement (41), das an der Befestigungskomponente montierbar ist und dazu konfiguriert ist, extern an der Haut des Patienten befestigt zu werden, um biologische Signale zu empfangen und diese biologischen Signale zu dem Adapterelement (3A, 3B) zu leiten; und
eine Einfassung (5) zum Einfassen des medizinischen Geräts (1) und des Adapterelements (3A, 3B) in einem montierten Zustand, in dem das Adapterelement (3A, 3B) an der mindestens einen Elektrode (130, 131) montiert ist.

## Revendications

1. Système de détection destiné à détecter des signaux biologiques de manière externe sur un patient, comprenant :
un dispositif médical implantable (1) comprenant au moins une électrode (130, 131) destinée à détecter des signaux biologiques sur un patient ;
un élément adaptateur (3A, 3B) pouvant être monté sur ladite au moins une électrode (130, 131) pour entrer en contact électrique avec l'au moins une électrode (130, 131) ;
un composant de fixation (4) pouvant être monté sur ledit élément adaptateur (3A, 3B) et conçu pour attacher le dispositif médical (1) de manière externe au patient ;
un élément de contact (41) pouvant être monté sur le composant de fixation (4) et conçu pour être attaché de manière externe à la peau du patient afin de recevoir des signaux biologiques et de conduire lesdits signaux biologiques jusqu'à l'élément adaptateur (3A, 3B) ; et
une enveloppe (5) destinée à envelopper le dispositif médical (1) et l'élément adaptateur (3A, 3B) dans un état monté dans lequel l'élément adaptateur (3A, 3B) est monté sur ladite au moins une électrode (130, 131).

2. Système de détection selon la revendication 1, dans lequel le dispositif médical (1) peut être implanté dans un patient, ladite au moins une électrode (130, 131) étant conçue pour entrer en contact électrique avec un tissu biologique afin de détecter des signaux biologiques dans un état implanté du dispositif médical (1).

3. Système de détection selon la revendication 1 ou 2, dans lequel le dispositif médical (1) est un biocapteur destiné à détecter des signaux d'électrocardiogramme.

4. Système de détection selon l'une des revendications 1 à 3, dans lequel le composant de fixation (4) peut être connecté de manière libérable audit élément adaptateur (3A, 3B).

5. Système de détection selon l'une des revendications précédentes, dans lequel au moins un parmi le dispositif médical (1) et l'élément adaptateur (3A, 3B) peut être retiré de l'enveloppe (5).

6. Système de détection selon l'une des revendications précédentes, dans lequel l'élément adaptateur (3A, 3B) peut être monté de manière libérable sur ladite au moins une électrode (130, 131).

7. Système de détection selon l'une des revendications précédentes, dans lequel l'élément adaptateur (3A, 3B) comprend un corps (30) formant une ouverture de réception (300) destinée à recevoir ladite au moins une électrode (130, 131) à l'intérieur de celle-ci.

8. Système de détection selon l'une des revendications précédentes, dans lequel le dispositif médical (1) comprend un boîtier (10) formant une partie de boîtier principale (100) et une partie de tête (101) s'étendant à partir de la partie de boîtier principale (100), et un module électronique (11) agencé dans ou sur la partie de boîtier principale (100).

9. Système de détection selon la revendication 8, dans lequel la partie de tête (101) s'étend linéairement depuis la partie de boîtier principale (100).

10. Système de détection selon la revendication 8 ou 9, dans lequel une première électrode (130) est placée sur la partie de tête (101) et une seconde électrode (131) est placée sur la partie de boîtier principale (100).

11. Système de détection selon la revendication 10, dans lequel un élément adaptateur (3A, 3B) peut être monté sur chacune parmi la première électrode (130) et la seconde électrode (131).

12. Système de détection selon l'une des revendications précédentes, dans lequel l'élément adaptateur (3A, 3B) et l'élément de fixation (4) sont formés d'une seule pièce.

13. Système de détection selon l'une des revendications précédentes, dans lequel l'élément adaptateur (3A, 3B), l'élément de fixation (4) et l'élément de contact (41) sont formés d'une seule pièce.

14. Procédé de fourniture d'un système de détection destiné à détecter des signaux biologiques de manière externe sur un patient, comprenant les étapes consistant à :
fournir un dispositif médical implantable (1) comprenant au moins une électrode (130, 131) destinée à détecter des signaux biologiques sur un patient ;
monter un élément adaptateur (3A, 3B) sur ladite au moins une électrode (130, 131) pour entrer en contact électrique avec l'au moins une électrode (130, 131) ;
fournir un composant de fixation (4) pouvant être monté sur ledit élément adaptateur (3A, 3B) et conçu pour attacher le dispositif médical (1) de manière externe au patient ;
fournir un élément de contact (41) pouvant être monté sur le composant de fixation et conçu pour être attaché de manière externe à la peau du patient afin de recevoir des signaux biologiques et conduire lesdits signaux biologiques jusqu'à l'élément adaptateur (3A, 3B) ; et
envelopper, en utilisant une enveloppe (5), le dispositif médical (1) et l'élément adaptateur (3A, 3B) dans un état monté dans lequel l'élément adaptateur (3A, 3B) est monté sur ladite au moins une électrode (130, 131).

15. Utilisation d'un système de détection destiné à détecter des signaux biologiques de manière externe sur un patient, le système de détection comprenant :
un dispositif médical implantable (1) comprenant au moins une électrode (130, 131) destinée à détecter des signaux biologiques sur un patient ;
un élément adaptateur (3A, 3B) pouvant être monté sur ladite au moins une électrode (130, 131) pour entrer en contact électrique avec l'au moins une électrode (130, 131) ;
un composant de fixation (4) pouvant être monté sur ledit élément adaptateur (3A, 3B) et conçu pour attacher le dispositif médical (1) de manière externe au patient ;
un élément de contact (41) pouvant être monté sur le composant de fixation et conçu pour être attaché de manière externe à la peau du patient afin de recevoir des signaux biologiques et conduire lesdits signaux biologiques jusqu'à l'élément adaptateur (3A, 3B) ; et
une enveloppe (5) destinée à envelopper le dispositif médical (1) et l'élément adaptateur (3A, 3B) dans un état monté dans lequel l'élément adaptateur (3A, 3B) est monté sur ladite au moins une électrode (130, 131).
